Europäisches Patentamt

European Patent Office

Office européen des brevets

(19)

(11) Numéro de publication : **0 500 477 A1**

## (12) DEMANDE DE BREVET EUROPEEN

(21) Numéro de dépôt : **92420050.4**

(22) Date de dépôt : **18.02.92**

(51) Int. Cl.⁵ : **A61F 2/34**

(30) Priorité : **19.02.91 FR 9102368**

(43) Date de publication de la demande :
**26.08.92 Bulletin 92/35**

(84) Etats contractants désignés :
**CH DE ES IT LI**

(71) Demandeur : **EUROS Société Anonyme
Z.E. Athelia III
F-13600 La Ciotat (FR)**

(71) Demandeur : **Scheiner, Jean Pierre
Place des Marronniers
F-13480 Cabries (FR)**

(72) Inventeur : **Schreiner, Jean Pierre
Place des Marronniers
F-13480 Cabries (FR)**

(74) Mandataire : **Dupuis, François et al
Cabinet Laurent et Charras, 3 Place de
l'Hôtel-de-Ville, BP 203
F-42005 St. Etienne Cédex 1 (FR)**

(54) **Implant cotyloidien à vis.**

(57)   Implant cotyloïdien comprenant un anneau métallique (1) recevant un noyau en matière plastique (4), l'anneau étant du type impacté à vis, caractérisé en ce que l'anneau (1) est d'épaisseur réduite et présente, dans la partie (1a) destinée à coopérer en appui au niveau du toit de la cavité cotyloïde, des moyens (2) aptes à recevoir des vis de fixation (3), de manière à ce que les têtes de vis ne débordent pas à l'intérieur dudit anneau, lesdits moyens (2) présentant une partie qui apparait en débordement de la périphérie externe de l'anneau (1) la partie débordante de ces moyens présentant des aspérités (2c) aptes à s'impacter dans le toit du cotyle.

FIG.4

De manière connue, ce type d'impant comprend un anneau métallique recevant un noyau, notamment en polyéthylène. Ce type d'implant est destiné à remplacer la cavité cotyloïde de l'os iliaque, dans laquelle s'articule la tête fémorale.

Plus particulièrement, l'invention concerne les implants cotyloïdiens dont l'anneau est fixé dans la cavité cotyloïde sans ciment. Notamment certains types d'anneaux sont conformés pour être impactés en force dans la cavité et peuvent présenter certains agencements pour assurer la fixation primaire. Le plus souvent, cette impaction primaire est complétée par une fixation complémentaire, généralement par vis.

La fixation par vis de l'anneau, nécessite de réaliser ce dernier dans une épaisseur relativement importante pour permettre d'effacer les têtes de vis, ce qui est nécessaire pour la mise en place du noyau, afin que ce dernier porte de manière régulière dans la cavité hémisphérique ou autres formes dudit anneau.

On conçoit que cette épaisseur, non seulement augmente le coût de fabrication, mais également augmente inutilement le poids.

Un des premiers problèmes que se propose de résoudre l'invention est de pouvoir exécuter un anneau d'épaisseur réduite, tout en permettant d'assurer sa fixation dans la cavité cotyloïde, au moyen de vis à grosse tête notamment. Compte-tenu de l'épaisseur réduite de l'anneau, il a été nécessaire de concevoir des agencements particuliers permettant de noyer les têtes de vis, afin de ne pas altérer le bon positionnement du noyau.

Le problème étant ainsi posé, on a voulu également améliorer la fixation primaire de l'anneau après impaction. Ce double problème est résolu en ce que l'anneau présente, dans la partie entrant en contact avec le toit du cotyle, des trous aptes à recevoir des bagues pour le logement de vis de fixation, lesdites bagues débordant de la périphérie externe de l'anneau, la partie débordante de ces bagues présentant des aspérités aptes à s'impacter dans le toit du cotyle.

Avantageusement, le problème posé d'intégrer les têtes de vis dans l'épaisseur réduite de l'anneau est résolu par le fait que chaque bague présente une colerette d'appui et une portée cylindrique engagée dans des trous formés dans l'épaisseur de l'anneau, les aspérités étant formées au niveau du bord circulaire de la portée cylindrique qui déborde de la périphérie de l'anneau.

Un autre problème que se propose de résoudre l'invention est d'améliorer la fixation primaire de l'anneau après impaction dans la cavité cotyloïde.

Pour résoudre ce problème, on a déjà proposé de former, en débordement de la périphérie de l'anneau, des pointes métalliques, picots ou autres aspérités d'ancrage. Généralement, ces picots sont régulièrement répartis sur la périphérie de l'anneau en étant situés seulement, au niveau de la partie périphérique ouverte dudit anneau.

Compte-tenu de la répartition des picots, il apparait que cette solution ne tiend pas compte des efforts subis par l'implant et des caractéristiques de la cavité cotyloïde qui présente, au niveau du toit, une zone de dureté supérieure.

Le problème que se propose de résoudre l'invention est d'assurer une parfaite stabilité primaire de l'anneau, notamment au niveau de la zone du toit de la cavité où les efforts appliqués sont les plus importants.

Un tel problème est résolu en ce que la partie de l'anneau recevant les bagues délimite une zone qui présente en débordement, des aspérités ou picots d'ancrage.

Avantageusement, pour parfaire la stabilité et en complément de la zone de l'anneau recevant les picots d'ancrage, ce dernier présente périphériquement, à proximité de son ouverture recevant le noyau, d'autres picots d'ancrage.

Pour résoudre le problème posé de faciliter l'impaction de l'anneau dans dans la cavité cotyloïde, ce dernier présente au moins une fente radiale formée depuis son rebord supérieur jusqu'au niveau de sa zone polaire qui est ouverte, sans toutefois être en communication avec l'ouverture de ladite zone.

Dans une forme de réalisation préférée, les fentes sont décalées de 120°, deux d'entre elles étant formées de part et d'autre de la zone recevant les picots.

D'une manière connue, ces fentes participent à la déformation par élasticité de l'anneau sous l'action d'un organe impacteur, en vue de son introduction dans la cavité cotyloïde pour assurer le blocage primaire. Ce blocage primaire est accentué par l'introduction d'un organe impacteur métallique, de diamètre et de dimensions identiques à ceux du noyau en polyéthylène, permettant ainsi la pénétration complète des pointes dans le cotyle.

Pour résoudre le problème posé d'assurer la fixation du noyau par rapport à l'anneau, ce dernier est agencé pour recevoir le noyau par déformation élastique.

Avantageusement, le problème posé de la fixation du noyau dans l'anneau est résolu en ce que le noyau présente un rebord circulaire apte à prendre appui sur le bord circulaire de l'anneau, la périphérie du noyau présente, sous ledit rebord et à proximité de ce dernier, une colerette apte à coopérer avec une gorge circulaire formée périphériquement dans la cavité hémisphérique de l'anneau.

L'invention est exposée ci-après plus en détail à l'aide des dessins annexés, dans lesquels :

La figure 1 est une vue en perspective de l'anneau selon l'invention.

La figure 2 est une vue en coupe partielle de l'anneau considéréé selon a ligne 2.2 de la figure 1.

La figure 3 est une vue en coupe partielle considérée selon la ligne 3.3 de la figure 1.

La figure 4 est à une échelle plus importante, une vue en coupe au niveau des bagues recevant les vis de fixation.

La figure 5 est à une échelle plus importante, une vue en coupe partielle, montrant la fixation du noyau dans l'anneau.

D'une manière parfaitement connue, l'implant cotyloïdien comprend un anneau (1) du type hémisphérique, exécuté en tout type de matériau, tel que titane et susceptible d'être soumis à tout traitement de surface approprié. Cet anneau (1) reçoit un noyau (4) en matière plastique, notamment en polyéthylène.

Suivant une première caractéristique à la base de l'invention, l'anneau (1) présente dans sa partie (1a) destinée à prendre appui, au niveau du toit de la cavité cotyloïde de l'os iliaque, des trous (1b) pour le passage d'organes de fixation.

Plus particulièrement, ces trous (1b) sont destinés à recevoir des bagues (2) conformées pour, d'une part, permettre le logement de vis de fixation (3) et, d'autre part, améliorer l'ancrage de l'anneau (1), dans la cavité cotyloïde.

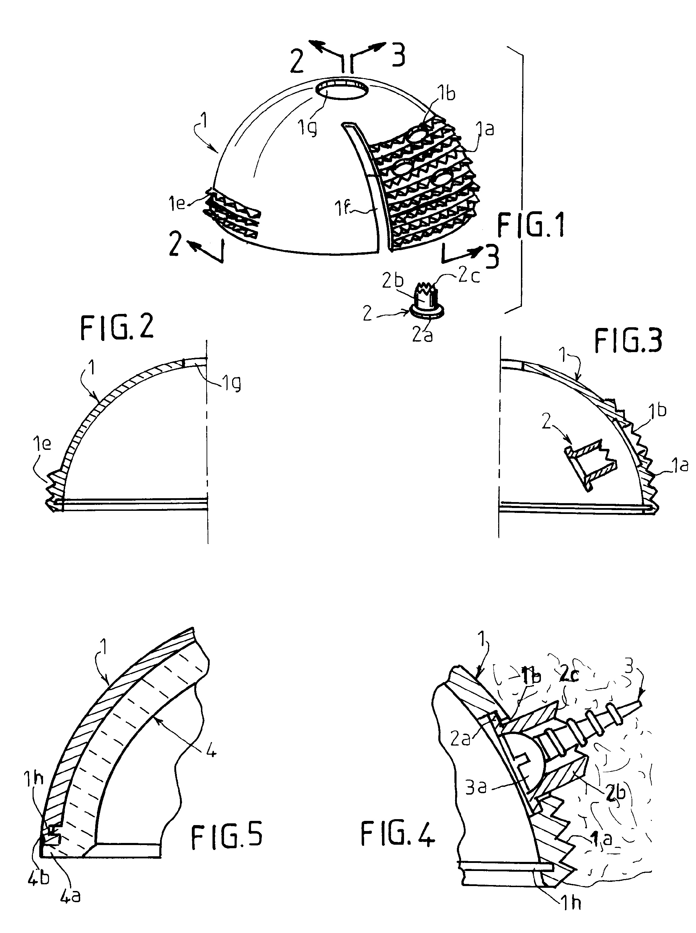

Comme le montrent notamment les figures 1, 3 et 4, chaque bague présente une colerette d'appui (2a) et une portée cylindrique (2b). La colerette (2a) coopère en appui dans une partie chanfreinée des trous (1b). La tête (3a) des vis (3) est positionnée et centrée à l'intérieur de la portée cylindrique de la bague (2).

Après engagement des bagues (2) dans les trous (1b), la portée cylindrique (2b) déborde de la périphérie externe de l'anneau pour être impactée au niveau du toit de la cavité cotyloïde. D'une manière importante, l'extrémité de la portée cylindrique (2b) présente, en bout, des aspérités d'ancrage (2c). Par exemple, ces aspérités (2c) peuvent être formées par un profil en dents de scie, établi dans le prolongement des génératrices de la portée cylindrique (2b).

De manière préférée, les trous (1b), dans lesquels sont engagées les bagues (2), sont au nombre de trois, en étant disposés très sensiblement selon les sommets d'un triangle équilatéral (figure 1).

Suivant une autre caractéristique importante de l'invention, la partie (1a) de l'anneau délimite une zone qui présente, en débordement, des picots d'ancrage (1d). Cette zone s'étend, sur très sensiblement le tiers de la surface hémisphérique de l'anneau, depuis son bord supérieur ouvert, jusqu'au niveau de son extrémité polaire. Par exemple, ces picots (1d) peuvent être constitués par des profils très sensiblement prismatiques. Il apparait donc que cette zone équipée des picots (1d), assure, après impaction de l'anneau, une très bonne stabilité primaire, étant donné que cette zone est située au niveau du toit de la cavité cotyloïde, où l'os est le plus dur.

Avantageusement, toujours pour améliorer la stabilité de l'anneau, ce dernier présente, périphériquement, à proximité de son ouverture recevant le noyau, d'autres picots d'ancrage (1e). Par exemple, ces picots (1e) peuvent être disposés selon deux ou trois rangées parallèles (figure 2).

L'anneau, selon l'invention, est notamment du type de ceux qui sont impactés à force, dans la cavité cotyloïde, après déformation élastique. Dans ce but, l'anneau (1) présente au moins une fente radiale (1f) formée depuis son rebord supérieur, jusqu'au niveau de sa partie polaire.

A noter que la partie polaire de l'anneau peut présenter une ouverture (1g) permettant, de manière connue, de visualiser le fond de la cavité cotyloïde et de favoriser l'élasticité de l'anneau.

De manière préférée, l'anneau présente trois fentes (1f) décalées de 120°, deux de ces fentes étant disposées très sensiblement de part et d'autre de la zone recevant les picots (1d). Dans ce cas, les fentes (1f), ne sont pas en communication avec l'ouverture (1g) de la zone polaire.

Suivant une autre caractéristique, la fixation du noyau (4), dans la cavité hémisphérique de l'anneau, s'effectue par déformation élastique. Comme le montre la figure 5, le noyau présente un rebord circulaire (4a), apte à prendre appui sur le bord circulaire de l'anneau (1). La périphérie du noyau (4) présente, à proximité du rebord (4a) et d'une manière parallèle à ce dernier, une colerette (4b), apte à être engagée dans une gorge circulaire (1h), formée périphériquement dans la cavité hémisphérique de l'anneau (1).

Les avantages ressortent bien de la description.

**Revendications**

-1- Implant cotyloïdien comprenant un anneau métallique (1) recevant un noyau en matière plastique (4), l'anneau étant du type impacté à vis, caractérisé en ce que l'anneau (1) est d'épaisseur réduite et présente, dans la partie (1a) destinée à coopérer en appui au niveau du toit de la cavité cotyloïde, des moyens (2) aptes à recevoir des vis de fixation (3), de manière à ce que les têtes de vis ne débordent pas à l'intérieur dudit anneau, lesdits moyens (2) présentant une partie qui apparait en débordement de la périphérie externe de l'anneau (1) la partie débordante de ces moyens présentant des aspérités (2c) aptes à s'impacter dans le toit du cotyle.

-2- Implant selon la revendication 1, caractérisé en ce que les moyens sont constitués par des bagues (2), engagées dans des trous (1b), formés dans l'épaisseur de la partie (1a) de l'anneau.

-3- Implant selon la revendication 2, caractérisé en ce que chaque bague (2) présente une colerette d'appui (2a) et une portée cylindrique (2b) engagée dans les trous (1b) formés dans l'épaisseur de l'anneau (1), les aspérités (2c) étant formées au niveau du bord circulaire de la portée cylindrique (2b) qui déborde de la périphérie de l'anneau.

-4- Implant selon la revendication 1, caractérisé en ce que la partie de l'anneau recevant les bagues (2) délimite une zone qui présente en débordement, des pointes d'ancrage (1d).

-5- Implant selon la revendication 4, caractérisé en ce que l'anneau présente périphériquement, à proximité de sa partie ouverte recevant le noyau (4), d'autres picots d'ancrage (1e).

-6- Implant selon la revendication 1, caractérisé en ce que l'anneau (1) présente au moins une fente radiale (1f) formée depuis son rebord supérieur jusqu'au niveau de la zone polaire qui est ouverte, sans toutefois être en communication avec l'ouverture (1g) de ladite zone.

-7- Implant selon la revendication 6, caractérisé en ce que les fentes (1f) sont décalées de 120°, deux d'entre elles étant formées de part et d'autre de la zone recevant les picots (1d).

-8- Implant selon la revendication 1, caractérisé en ce que l'anneau (1) est agencé pour recevoir le noyau (4) par déformation élastique.

-9- Implant selon la revendication 8, caractérisé en ce que le noyau (4) présente un rebord circulaire (4a) apte à prendre appui sur le bord circulaire de l'anneau (1), la périphérie du noyau présente, sous ledit rebord et à proximité de ce dernier, une colerette (4b) apte à coopérer avec une gorge circulaire (1h) formée périphériquement dans la cavité hémisphérique de l'anneau.

FIG.1

FIG.2

FIG.3

FIG.5

FIG.4

Office européen
des brevets

**RAPPORT DE RECHERCHE EUROPEENNE**

Numero de la demande

EP    92 42 0050

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 341 199 (GEBRÜDER SULZER AG) | 1 | A61F2/34 |
| A | * colonne 2, ligne 22 - ligne 26; figure 1 * | 2-4 | |
| | --- | | |
| Y | WO-A-8 502 535 (PROTEK AG) | 1 | |
| A | * figures 6,7 * | 8,9 | |
| | --- | | |
| A | DE-A-3 310 944 (ERLER) | 1,8 | |
| | * figures 8,9 * | | |
| | --- | | |
| A | EP-A-0 225 819 (DUTHOIT ET AL) | 1,6 | |
| | * figures 1,5 * | | |
| | --- | | |
| A | EP-A-0 333 642 (GEBRÜDER SULZER AG) | 1 | |
| | * revendications 1,3; figure 1 * | | |
| | ----- | | |
| | | | **DOMAINES TECHNIQUES RECHERCHES (Int. Cl.5)** |
| | | | A61F |

Le présent rapport a été établi pour toutes les revendications

| Lieu de la recherche | Date d'achèvement de la recherche | Examinateur |
|---|---|---|
| BERLIN | 19 MAI 1992 | KANAL P. |

EPO FORM 1503 03.82 (P0402)